## Europäisches Patentamt

## European Patent Office

## Office européen des brevets

(11) Publication number: **0 279 704**
**A2**

(12) **EUROPEAN PATENT APPLICATION**

(21) Application number: **88301454.0**

(22) Date of filing: **19.02.88**

(51) Int. Cl.⁴: **A 01 N 35/02**
**A 01 N 59/26**
**//(A01N35/02,31:08),**
**(A01N59/26,35:02,31:08)**

(30) Priority: **19.02.87 US 16348   25.02.87 US 18516**

(43) Date of publication of application:
**24.08.88   Bulletin 88/34**

(84) Designated Contracting States:
**BE DE FR GB IT NL**

(71) Applicant: **HEALTH CARE PRODUCTS, INC.**
**260 "B" Brunel Road**
**Mississauga Ontario L4Z 1T5 (CA)**

(72) Inventor: **Strong, Frank S.**
**1050 Shawnmarr**
**Mississauga Ontario (CA)**

(74) Representative: **Ablewhite, Alan James et al**
**MARKS & CLERK 57/60 Lincoln's Inn Fields**
**London WC2A 3LS (GB)**

(54) Santized, sterilized, disinfected and sporicidal articles and processes for sanitizing, sterilizing, disinfecting and rendering objects sporicidal and improved sanitizing, sterilizing, and desinfecting composition.

(57) A process for sanitizing, sterilizing disinfecting, and killing spores involves providing a tangible object made from polymeric material impregnated with an aqueous glutaraldehyde solution which is packaged in an air-tight enclosure, removing the impregnated tangible object from the enclosure, and applying the impregnated tangible object to the surface to be sterilized and/or sanitized and disinfected so as to expose spores, bacteria, virus, and other microorganisms present on the surface to the action of the glutaraldehyde solution. A sanitary attachment for a receiver or mouthpiece of a telephone made from plastic material incorporating a glutaraldehyde solution. A fabric made from cellulosic or plastic material having sporicidal and/or sanitizing, sterilizing and disinfectant activity as a result of being impregnated with a glutaraldehyde solution. A method of sanitizing, sterilizing and disinfecting and/or rendering fabric sporicidal involving impregnating a fabric with an aqueous glutaraldehyde solution prior to packaging the fabric in an air-impervious container until ready for use.

A sanitizing, sterilizing and disinfecting solution including an aqueous glutaraldehyde solution, phenol, monopotassium phosphate, disodium phosphate, sodium lauryl sulfate, and glycerol, preferably wherein glutaraldehyde is present in the amount 0.15%, phenol is present in the amount of 0.5%, monopotassium phosphate is present in the amount of 0.0388%, disodium phosphate is present in the amount of 1.108%, sodium lauryl sulfate is present in an amount of 0.25%, and glycerol is present in the amount of 0.5% with the remainder being water.

**Description**

**SANITIZED, STERILIZED, DISINFECTED AND SPORICIDAL ARTICLES, AND PROCESSES FOR SANTIZING, STERILIZING, DISINFECTING AND RENDERING OBJECTS SPORICIDAL AND IMPROVED SANITIZING, STERILIZING AND DISINFECTING COMPOSITION**

BACKGROUND OF THE INVENTION

1. Field of the Invention
The present invention is directed to sanitized, sterilized, disinfected and sporicidal articles and methods for manufacturing such articles. More particularly, the present invention is directed to fabrics and plastic articles incorporating aqueous sanitizing, sterilizing, and disinfecting compositions including glutaraldehyde. Specifically, the present invention is directed a sanitary attachment to be attached to a telephone receiver or mouthpiece formed from a plastic material and fabrics made from cellulosic or plastic materials incorporating aqueous sanitizing, sterilizing and disinfectant solutions including glutaraldehyde, sodium lauryl sulfate, glycerol, phenol, monosodium phosphate, and disodium phosphate.

The present invention is also directed to methods for manufacturing the acticles incorporating glutaraldehyde solutions and the method for sanitizing, sterilizing and disinfecting a contaminated surface using articles incorporating such glutaraldehyde solutions.

In addition, the present invention relates to improved disinfecting and sterilizing solution, and more particularly to disinfecting and sterilizing solutions having a composition containing aqueous glutaraldehyde solutions which are formulated to exhibit extended storage stability relative to conventional glutaraldehyde solutions. In particular, the present invention is directed to an improved chemical sterilizing and disinfecting solution having a composition containing glutaraldehyde, phenol, monopotassium phosphate and disodium phosphate, sodium lauryl sulfate and glycerol.

2. Discussion of Background and Material Information
The oldest and one of the most commonly used methods for disinfection has been sterilization using either boiling water or steam. In the past numerous compositions have been proposed for chemically disinfecting or sterilizing a variety of objects. Formaldehyde is one of the oldest chemosterilizers employed for the destruction of spores. The fumes of formaldehyde, however, limit its usefulness and its toxicity for tissue requires that disinfected materials be thoroughly rinsed with sterile water before use.

In addition, alcohols have also long been recognized as being effective sanitizing, sterilizing and disinfecting agents. A particularly suitable dialdehyde which has found considerable commercial success as a chemical sterilant is glutaraldehyde. Nevertheless, in order to render glutaraldehyde effective for this purpose, it must be alkalinated by being buffered with an appropriate agent effective for such purposes. Conventional alkaline solutions of glutaraldehyde as sterilizing agents, however, have been found to suffer from a number of disadvantages. A particular disadvantage of alklinated glutaraldehyde solutions is their relatively short shelf life.

Recently, alkalinized glutaraldehyde solutions have become widely used for such purposes. Typically, they consist of an aqueous glutaraldehyde solution buffered by suitable alkalinating agents to a pH 7.5 to 8.5. In the acid state at room temperature, glutaraldehyde solutions are stable for long periods of time when stored in a closed container. When rendered alkaline, however, the glutaraldehyde gradually undergoes polymerization and loses its activity, which proceeds very rapidly above pH 9.

Prior art attempts to remedy various problems associated with chemical disinfectants are documented in numerous patents. Representative examples of patents which relate to improved chemical sterilization and disinfecting compositions, and particularly to solutions containing saturated dialdehydes, such as glutaraldehyde, are discussed below.

U.S. Patent 3,016,328 is directed to a disinfecting composition containing a saturated dialdehyde, such as gutaraldehyde, and an alkalinating agent, in either alcoholic solution, or in aqueous solution, at above pH 7.4. The improvement of this patent resides in the discovery that a saturated dialdehyde containing 2 to 6 carbons has improved activity when it is combined with a lower alkanol and an alkalinating agent.

U.S. Patent 3,282,775 is directed to a disinfecting composition containing a $C_2$ to $C_6$ saturated dialdehyde, such as glutaraldehyde, and a cationic surface active agent. This patent teaches that the combination of saturated dialdehydes and cationic surface active agents results with potent activity and an extremely fast rate of killing a broad spectrum of microorganisms.

U.S. Patent 3,697,222 is directed to enhancing the activity of aqueous acid glutaradlehyde solutions at temperatures above 45°C by simultaneously subjecting such solutions to ultrasonic energy.

The use of ultrasonic energy has also found application in a number of other attempts to improve the efficacy of glutaraldehyde solutions. Representative examples of such patents include U.S. Patents 3,708,263; 3,912,450; 3,968,248; and 3,968,250.

U.S. Patent 3,912,450 also discloses that glutaraldehyde solutions combined with nonionic or anionic agents, such as ethoxylates of isomeric linear alcohols, or alkyl aryl sulfonates, are effective at a pH range 1 to 9 at temperatures within the range of 15°C to 75°C.

U.S. patent 3,917,850 is directed to a biocidal composition for surface and space disinfection which is composed of an aldehyde and/or dialdehyde, and a phenol derivative in a solvent. The composition preferably also includes an anionic surface active agent. It is disclosed that the phenol derivative can be substituted or unsubstituted and may be homologs of phenols, quinolinols and naphthols and their salts alone or in mixtures.

U.S. Patent 3,983,252 is directed to disinfectant compositions containing a dialdehyde and an alkali metal salt of a hydrocarbon carboxylic acid in an aqueous or alcohol solution. An alcohol solution including ethylene glycol, propylene glycol and butylene glycol and/or glycerol is disclosed as having improved stability at a pH 6.0 to 7.4.

U.S. Patent 4,048,336 is directed to a composition including glutaraldehyde and a monoaldehyde, such as formaldehyde, which is disclosed as exhibiting more rapid and effective antimicrobial activity than compositions containing either glutaraldehyde or the monoaldehyde alone.

U.S. Patent 4,093,744 is directed to a composition including glutaraldehyde and a detergent selected from the group of nonionic, anionic and ampholytic surface active agents. It is disclosed that the activity of the glutaraldehyde is enhanced by the inclusion of the detergents.

U.S. Patent 4,103,001 is directed to an aqueous sterilizing composition including glutaraldehyde, phenol and a metal phenate which is active at room temperature. The composition also includes sodium tetraborate, a humectant, such as glycerol, di-ethylglycerol, or propylene glycol, and a surfactant. The composition is formulated to have a pH within the range of 7.0-7.4.

U.S. Patent 4,436,754 is directed to a sterilizing and disinfecting composition which contains a dialdehyde formulated to exhibit lower odor and irritant potential by including a diol or a mono-substituted diol. The compositions may also contain other diols, such as ethylene glycol, propylene glycol, butylene glycol or triols, such as glycerol.

Of the foregoing, a buffered phenol-glutaraldehyde sterilizing composition which has met with wide acceptance is known commercially under the trade name Sporicidin and is the subject of U.S. Patent 4,103,001. The aqueous sporicidal composition of U.S. Patent 4,103,001 includes by weight from 0.75-4.0% glutaraldehyde and from 4-15% of a mixture of phenol and a metal phenate, the phenol content being from 3-10% and the metal phenate being from 0.5-5%, with the composition having ph of 7-10 and an active disinfecting life of at least 30 days. The sporicidal composition of U.S. Patent 4,103,001 was formulated in an attempt to overcome the disadvantages, particularly the limited stability, of alkaline glutaraldehyde solutions. The buffered phenol-glutaraldehyde combination is normally used by applying the solution to the surface of the object to be sterilized.

Fabrics have been produced which exhibit anti-bacterial and germicidal properties, for example, by the process of U.S. Patent 3,317,376 wherein fabrics are impregnated with an aqueous solution including from 0.8% to 15% by weight sodium phenolate, from 0.3% to 5.5% of sodium tetraborate, from 0.8% to 15% glycerine, from 2.0% to 16.5% of phenol and the balance water.

More recently, it has been suggested in U.S. Patent 4,446,967 to impregnate a germicide enclosure for dental and medical instruments made of a relatively thick layer of compressible absorbent material, such as foamed plastic material, with Sporicidin being mentioned as a suitable germicide for this purpose.

U.S. Patent 4,069,307 relates to a drug-delivery device for releasing a drug at a continuous and controlled rate for a prolonged period of time made from polymeric material such as an ethylene-vinyl acetate.

U.S. Patent 3, 317,376 is directed to a process for impregnating or otherwise treating fabrics with are made from synthetic or natural fibers with an aqueous solution of 0.8% to 15% by weight of sodium phenolate, 0.3% to 5.5% by weight of sodium tetraborate, 0.8% to 15% by weight of glycerine, 2.0% to 16.5% by weight of phenol.

U.S. Patent 1,741,668 is directed to a sanitary attachment made of porous material adapted to be connected to a telephone transmitter which is treated with a germicidal agent, such as formaldehyde.

Prior to the present invention, however, none of the prior art attempts to formulate effective sanitizing, sterilizing and disinfecting compositions have included aqueous glutaraldehyde solutions in combination with phenol, monopotassium phosphate, disodium phosphate, sodium lauryl sulfate, and glycerol, nor have conventional glutaraldehyde solutions exhibited the extent of storage stability possessed by sanitizing, sterilizing and disinfecting compositions comprising aqueous glutaraldehyde solutions, phenol, monopotassium phosphate, disodium phosphate, sodium lauryl sulfate, and glycerol in accordance with the present invention, nor have such glutaraldehyde solutions been impregnated into fabrics made from natural and synthetic materials which are formed into desired articles, such as sanitary towelettes and sanitary attachments for telephones, which are packaged in air-impervious containers until ready for use.

## SUMMARY OF THE INVENTION

An object of the present invention is a process for sanitizing, sterilizing disinfecting and killing bacteria, virus, spores and other microorganisms which involves providing a tangible object made from polymeric material which is impregnated with an aqueous glutaraldehyde solution and packaged in an air-tight enclosure; removing the impregnated tangible object from the enclosure; and applying the impregnated tangible object to inanimate surfaces so as to expose bacteria, virus and spores present thereon to the action of glutaraldehyde. The polymeric material is preferably selected from the group consisting of cellulosic materials and plastic materials, such as an ethylene-vinyl acetate resin, and as well as spunbonded olefins composed of high density polyethylene fibers which is commerically available as "TYVEX" (a registered brand) sold by

3

0 279 704

DuPont, with the tangible objects including appliances, bedding, cloths, clothing, containers, coverings, equipment, fabrics, flooring, garments, instruments, masks, towels, towelettes and utensils. Particularly preferred tangible objects for purposes of this process of the present invention are attachments to be connected to a telecommunication receiver or mouthpiece, and towelettes and wick dispensers for wiping a contaminated object.

Another object of the present invention is to provide a sanitary attachment to be attached to a receiver or mouthpiece of a piece of telecommunication equipment, such as a telephone, including a body portion of plastic material, such as TYVEX, incorporating a glutaraldehyde solution, and preferably also including a litmus base indicator, which is packaged in an air-impervious film.

Another further object of the present invention is the provision of a fabric having sanitizing, sterilizing disinfecting and sporicidal activity as a result of being impregnated with a glutaraldehyde solution, and preferably a litmus base indicator, which is packaged in an air-impervious container, wherein the fabric is made from a member selected from the group consisting of a cellulosic material and a plastic material.

Another still further object of the present invention is a method for sanitizing, sterilizing and disinfecting a fabric which involves impregnating fabric with an aqueous solution including glutaraldehyde prior to packaging the fabric in an air-impervious container until ready for use. The fabric is preferably also impregnated with an effective amount of a litmus base indicator.

It is also an object of the present invention is to provide a sterilizing and disinfecting composition, which is preferably bottled more preferably as a high level disinfectant formulation adapted to be sprayed directly onto target surfaces of tangible objects to be sterilized and disinfected, including a dialdehyde solution, phenol, at least one alkali metal salt of phosphoric acid, at least one surfactant and at least one humectant, preferably wherein the alkali metal salt of phosphoric acid is a member selected from the group consisting of potassium phosphates, sodium phosphates and mixtures of potassium phosphates and sodium phosphates, more preferably wherein the member of potassium phosphates is monopotassium phosphate and the member of sodium phosphates is disodium phosphate, and most preferably wherein the alkali metal salt is a mixture of monopotassium phosphate and disodium phosphate.

It is another object of the present invention to provide a sanitizing, sterilizing and disinfecting composition, as previously described, wherein the dialdehyde solution is an aqueous glutaraldehyde solution, the surfactant is sodium lauryl sulfate, and the humectant is glycerol.

It is another further object of the present invention to provide a santizing, sterilizing and disinfecting composition, as previously described, wherein the glutaraldehyde solution is a member selected from the group consisting of a 25% aqueous glutaraldehyde solution and a 50% aqueous glutaraldehyde solution, preferably wherein glutaraldehyde is present within the range of 0.05-0.5%, phenol is present within the range of 0.25-0.75%, monopotassium phosphate is present within the range of 0.01-0.075%, disodium phosphate is present within the range of 0.05-0.2%, sodium lauryl sulfate is present within the range of 0.1-0.3%, and glycerol is present within the range of 0.25-0.75% with the remainder being water.

It is another still further object of the present invention to provide a santizing, sterilizing and disinfecting composition, as previously described, wherein the glutaraldehyde is present within the range of 0.075- 0.3%, the phenol is present in the amount of about 0.5%, the monopotassium phosphate is present in the amount of about 0.0388%, the disodium phosphate is present in the amount of about 0.108%, the sodium lauryl sulfate is present in an amount of about 0.25%, and the glycerol is present in the amount of about 0.5%.

It is another yet still further object of the present invention to provide a sanitizing, sterilizing and disinfecting composition composed of 0.15% glutaraldehyde, 0.5% phenol, 0.0388% monopotassium phosphate, 0.108% disodium phosphate, 0.25% sodium lauryl sulfate, and 0.5% glycerol with the remainder being water.

Another object of the present invention is to provide a composition for activating dialdehyde solutions in the production of sanitizing, sterilizing and disinfecting compositions wherein the composition for activating is composed of phenol and at least one alkali metal salt of phosphoric acid, preferably wherein the alkali metal salt of phosphoric acid is a member selected from the group consising of potassium phosphates, sodium phosphates, and mixtures of potassium phosphates and sodium phosphates, more preferably wherein the alkali metal salt of phosphoric acid is a member selected from the group consisting of monopotassium phosphate and disodium phosphate, and most preferably is a mixture of monopotassium phosphate and disodium phosphate.

Another further object of the present invention is to provide a composition for activating dialdehyde solutions, as previously described, which further includes at least one surfactant and at least one humectant, wherein the surfactant is sodium lauryl sulfate, and the humectant is glyerol.

Another still further object of the present invention is to provide a composition for activating dialdehyde solutions, as previously described, consisting essentially of phenol, monopotassium phosphate, disodium phosphate, sodium lauryl sulfate, glycerol and water.

It is another object of the present invention to provide a method for producing a santizing, sterilizing and disinfecting glutaraldehyde solution having extended shelf life which involves providing a composition including phenol, monopotassium phosphate, disodium phosphate, sodium lauryl sulfate, glycerol and water; mixing 15% by total weight of a glutaraldehyde solution with 85% by total weight of the previously described composition to result with a sanitizing, sterilizing and disinfecting glutaraldehyde solution composed of 0.05-0.5% by total weight glutaraldehyde, 0.5% by total weight phenol, 0.0388% by total weight monopotassium phosphate, 0.108% by total weight disodium phosphate, 0.25% by total weight sodium lauryl

4

sulfate, 0.5% by total weight glycerol, and the balance being solvent.

An object of the present invention is a new sanitizing, sterilizing and disinfecting composition composed of an aqueous glutaraldehyde solution, phenol, monopotassium phosphate, disodium phosphate, sodium lauryl sulfate and glycerol, preferably composed of 0.05-0.5% of glutaraldehyde, 0.25-0.75% phenol, 0.01.-0.075% monopotassium phosphate, 0.05-0.2% disodium phosphate 0.1-0.3% sodium lauryl sulfate, and 0.25-0.75% glycerol, and most preferably composed of 0.075-0.3% of 25%-50% aqueous glutaraldehyde solution, 0.5% phenol, 0.03% monopotassium phosphate, 0.108% disodium phosphate, 0.25% sodium lauryl sulfate and 0.5% glycerol.

## BRIEF DESCRIPTION OF THE DRAWINGS

Fig. 1 is a plane view of a telephone disk for attachment to a telephone transmitter or receiver;

Fig. 2 is a cross-section view of the telephone disk of Fig. 1 taken along line II-II; and

Fig. 3 is a side view of a package containing articles made in accordance with the present invention;

Fig. 4 is a plan view of another embodiment of a telephone disk for attachment to a telephone transmitter or receiver; and

Fig. 5 is a cross-sectional view of the telephone disk of Fig. 4 taken along line V-V.

## DETAILED DESCRIPTION

In accordance with the present invention, it has been discovered that numerous items, including fabrics and plastic articles, may be impregnated with solutions comprising glutaraldehyde, sodium lauryl sulfate glycerol, phenol, monosodium phosphate, and disodium phosphate and solutions comprising glutaraldehyde and a mixture of phenol and metal phenate, to render them sporicidal and/or sanitized, sterilizing and disinfected for extended periods of time. As previously mentioned, solutions including glutaraldehyde have been found to be very effective against a wide variety of microorganisms including virus, such AIDS and Herpes. Although articles and fabrics made from cellulosic and plastic material have been impregnated with a variety of active agents, including germicidal solutions, so as to deliver the active agent to a desired target over a prolonged period of time, it is not believed that aqueous glutaraldehyde solutions comprising glutaraldehyde, sodium lauryl sulfate glycerol, phenol, monosodium phosphate, and disodium phosphate and aqueous solutions comprising glutaraldehyde and a mixture of phenol and metal phenate have been effectively used for this purpose. Notwithstanding the improvements made to the storage stability of glutaraldehyde solutions as set forth in U.S. Patent 4,103,001, even Sporicidin lacks the proper storage stability when exposed to the atmosphere to be generally useful in delayed delivery systems. In accordance with the present invention, therefore, articles incorporating aqueous glutaraldehyde solutions comprising glutaraldehyde, sodium lauryl sulfate glycerol, phenol, monosodium phosphate, and disodium phosphate and aqueous solutions comprising glutaraldehyde and a mixture of phenol and metal phenate are individually packaged in air-tight containers and stored until ready for use. As desired, the particular article is then removed from its air-tight container and used in accordance with the particular application for which it was intended.

The present invention may make use of any of the commercially available glutaraldehyde solutions, such as those which are currently marketed under the brand names Cidex, Sonacide, Sporicidin and Ucarcide.

Of the previously mentioned commercially available glutaraldehyde solutions, glutaraldehyde solutions which function effectively for purposes of the present invention include buffered phenol-glutaraldehyde sterilizing compositions made in accordance with U.S. Patent 4,103,001. Sporicidin has been found to be effective for purposes of the present invention because it exhibits a pH of about 7-10 and an active sterilizing life of at least 30 days. The glutaraldehyde solution commercially sold as Sporicidin is an aqueous composition containing from 0.75-4.0% glutaraldehyde and from 4-15% of a mixture of phenol and a metal phenate, with the phenol content being from 3-10% and the metal phenate being from 0.5-5% by weight of the composition. The composition preferably also includes 1-5% by weight of sodium tetraborate and preferably at least one anionic or non-ionic surfactant, which may be selected from the group consisting of sodium dodecyl benzene sulphonate and sodium cocoyl sarcosinate, preferably in the amount of 2-10% by the weigh of composition, as well as a humectant, selected from the group consisting glycerol di-ethylene glycol and propylene glycol. The preferred ratio of phenol to sodium phenate in the aqueous composition is 5-7 to 1.

The glutaraldehyde solutions which have been found to be particularly effective for purposes of all embodiments of the present invention, however, are aqueous solutions selected from the group of solutions consisting of solutions comprising glutaraldehyde, sodium lauryl sulfate glycerol, phenol, monosodium phosphate, and disodium phosphate, and solutions comprising glutaraldehyde and a mixture of phenol and metal phenate. More preferred glutaraldehyde solutions for purposes of the present invention are members selected from the group of solutions consisting of solutions comprising 0.005-0.30% glutaraldehyde, 0.1-0.3% sodium lauryl sulfate, 0.25-0.75% glycerol, 0.25-0.75% phenol, 0.01-0.03% monopotassium phosphate, and 0.1-0.12% disodium phosphate, and solutions comprising 0.75-4.0% glutaraldehyde and from 4-15% of a mixture of phenol and metal phenate, with the phenol content being from 3-10% and the metal phenate being from 0.5-5% by weight of said aqueous solution. The most preferred solution for purposes of the present invention comprises 0.75-0.15% glutaraldehyde, 0.25% sodium lauryl sulfate, 0.5% glyercol, 0.5% phenol, 0.03% monopotassium phosphate, and 0.108% disodium phosphate.

The present invention, therefore, is also based on the discovery that a sanitizing, sterilizing and disinfecting composition containing aqueous glutaraldehyde solutions can be formulated so as to overcome many of the disadvantages experienced by glutaraldehyde solutions previously discussed. In this regard, the aqueous glutaraldehyde solution of the present invention does not exhibit strong objectionable odor, is equivalently fast acting, and is stable for longer periods of time, relative to conventional formulations of glutaraldehyde solutions.

Glutaraldehyde is typically commerically available as an aqueous solution, normally containing between 25-50% glutaraldehyde. As previously mentioned, and as is well known in the art, aqueous glutaraldehyde autopolymerizes irreversably under alkaline conditions which ultimately results with a loss of biological activity. For example, at pH 8.5, 2% glutaraldehyde maintains its germicidal and sporicidal activity normally for no longer than about about two weeks. The prior art attempts to buffer glutaraldehyde to a pH 7.5-8.0 have been somewhat effective in extending the storage stability for slightly longer periods of time. It is generally recognized, however, that the buffered phenol-glutaraldehyde sterilizing compositions, such as the Sporicidin brand sterilizing composition covered by U.S. Patent 4,103,001 which is slightly alkaline at pH 7.0-7.4, has the longest shelf life of up to about 30 days.

An unexpected discovery of the present invention is the provision of a sanitizing, sterilizing and disinfecting composition containing aqueous glutaraldehyde solutions which has an extended storage stability of up to 60 days after the aqueous glutaraldehyde solution has been mixed with an activating agent composition, including the alkalinizing agent.

Although glutaraldehyde is the preferred dialdehyde for purposes of the present invention, it is believed that the advantages of the present invention may also be realized by formulating sanitizing, sterilizing and disinfecting compositions including saturated dialdehydes such as those containing from 2 to 6 carbon atoms, in addition to glutaraldehyde, otherwise in accordance with the present invention. Furthermore, the dialdehydes which may be used for purposes of the present invention may be in their basic form, or in the form of an adduct or in other forms well-known in the art. Unless otherwise indicated, however, glutaraldehyde shall be used for purposes of the discussions herein, and the example presented herein is directed to the use of glutaraldehyde.

For purposes of formulating the improved sanitizing, sterilizing and disinfecting composition of the present invention, the source of glutaraldehyde is preferably selected from one of the commercially available glutaraldehyde solutions, such as those which are currently marketed under the brand names Ucarcide and Glutaraldehyde Aqueous Grade 2. The most preferred commercially available form of glutaraldehyde is Ucarcide manfactured and sold by the Union Carbide Company. The Ucarcide brand glutaraldehyde solution is available in 25% and 50% aqueous solutions, either of which is suitable for purposes of the present invention taking into consideration use of the appropriate amount so as to result with a final formulation in accordance with the claims presented herein. In this regard, the amount of glutaraldehyde solution which may be used in the composition of the present invention may vary between 0.05-0.5% by total weight of the final sanitizing, sterilizing and disinfecting solution, although the range of 0.075-0.3% by total weight is preferred, and the use of 50% aqueous glutaraldehyde solutions in an amount sufficient to provide 0.15% glutaraldehyde by total weight of the final sanitizing, sterilizing and disinfecting compositions yields the most preferred results. Depending on whether a 25% or 50% aqueous solution of glutaraldehyde is used, the final sanitizing, sterilizing and disinfecting solution prepared in accordance with the present invention will include preferably between 0.05% and 0.5% by total weight glutaraldehyde.

As previously mentioned, the addition of activating agents, for example to adjust the pH of the aqueous glutaraldehyde solutions, is well known to those skilled in the art. Conventionally, glutaraldehyde solutions have been found to be most stable within the pH range of about 2.5-4.5% which is the pH level at which they are normally stored before use. Prior to use, their pH levels are adjusted by the addition of various activating agents such as the ones disclosed in the prior art. As previously mentioned, the activating agents which have been proposed are as varied as the effects they are purported to achieve. In any event, glutaraldehyde solutions are conventionally manufactured as a two-package system. One container includes glutaraldehyde in a suitable solvent, and another container packages the activating agents, which also are normally in solution. Typically, the appropriate amounts of the glutaraldehyde solution and the solution of activating agents have been premeasured so that it is a relatively simple matter to mix one with the other to activate the glutaraldehyde for use. Once activated in this manner, however, the glutaraldehyde compositions of the prior art must be used relatively quickly for their intended purpose inasmuch as they are generally recognized having limited shelf lives.

The present invention is based on the discovery that activating aqueous glutaraldehyde solutions by mixing with the novel and unique activating agent composition of the present invention maintains the efficacy of the final sanitizing, sterilizing and disinfecting solution containing the glutaraldehyde for extended periods of time.

An important ingredient for purposes of the activating agent composition of the present invention is phenol. It has been discovered that phenol must be present in amounts within the range of about 0.25-0.75% by total weight of the final sanitizing, sterilizing and disinfecting composition in order for the advantages in accordance with the present invention to be realized. Preferably, the phenol should be present in the amount of about 0.5% by total weight of the final sanitizing, sterilizing and disinfecting composition. The phenols useful for purposes of the present invention also include phenol derivatives which have one or several phenol functions and may be simple phenol homologs, or halogenated phenol derivatives. Although homologs such as di or tri hydric phenols, nitrophenols, amino phenols, quinolinols, naphthols, and the like may be useful for purposes of the present invention, the preferred phenol analogues are those manufactured and sold under the brand name

6

Dowicide. Thus, the preferred phenol analogues for purposes of the present invention include ortho-phenylphenol (Dowicide 1) and a solution of ortho-benzyl-perachlorophenol in isopropyl alcohol (Dowicide OBCP-IPA), with Dowicide 1 being most preferred.

In addition to phenol, it is also important that the activating agent composition include alkali metal salts of phosphoric acid so as to effect the required buffering of the phenol-glutaraldehyde combination in the final composition. In this regard, the alkali metal salts of phosphoric acid may be employed in amounts within the range of about 0.07-0.25% by total weight of the final sanitizing, sterilizing and disinfecting composition. The alkali metal salts of phosphoric acid which have been found to be particularly suitable for purposes of the present invention include potassium and sodium salts of phosphoric acid. In this regard, monopotassium phosphate and disodium phosphate are preferred and it has been discovered a mixture of alkali metal salts of phosphoric acid, particularly monopotassium phosphate and disodium phosphate yields particularly preferred results. In such instance, monopotassium phosphate is preferably present within the range of 0.01-0.075% and disodium phosphate is present within the range of 0.05-0.2% by total weight of the final sanitizing, sterilizing and disinfecting composition. Most preferably, monopotassium phosphate is present in an amount of about 0.0388% and disodium phosphate is present in the amount of 0.108% by total weight of the sanitizing, sterilizing and disinfecting composition.

Although not wishing to be bound by any particular theory, it is believed that the use of alkali metal salt of phosphoric acid, and specifically the use of a mixture of monopotassium phosphate and disodium phosphate in the previously mentioned amounts, contributes to resulting with a final sanitizing, sterilizing and disinfecting composition including an aqueous glutaraldehyde solution which is active for almost twice as long as prior art phenol-glutaraldehyde combinations, for example buffered with sodium phenate as disclosed by U.S. Patent 4,103,001.

In addition to the phosphate buffering agents, the activating agent composition used in accordance with the present invention is also composed of surfactants and humectants.

Although it is believed that alcohols, including lower alkanols containing up to and including about seven carbon atoms, in addition to alkanediols containing from two to four carbons and the like, could be used as humectants to formulate the activating agent composition used to activate sanitizing, sterilizing and disinfecting solutions containing aqueous glutaraldehyde solutions, it has been found that alkanetriols, such as glycerol, are preferred in producing the most effective results for purposes of the present invention. In this regard glycerol is employed in an amount to provide a final concentration in the sanitizing, sterilizing and disinfecting composition within the range of 0.25-0.75% by total weight of the composition, and more preferably is present in the amount of 0.5% by total weight of the composition.

Also, the activating agent composition used in accordance with the present invention should contain surfactants, and preferably sodium lauryl sulfate, in sufficient amounts to function in various ways to increase the effectiveness of the sanitizing, sterilizing and disinfecting solution. In this regard, sodium lauryl sulfate should be present in the range of 0.1-0.3% and is preferably present in the amount of 0.25% by total weight of the final sanitizing, sterilizing and disinfecting solution.

Other substances may also be added to the activating agent composition or to the final sanitizing, sterilizing and disinfecting compositions of the present invention provided that they have no deleterious effect on the sanitizing, sterilizing and disinfecting activity of the final solution. Representative samples of the such substances include coloring materials, pH indicators, buffers, anti-corrosion agents, dyes and the like.

Although the solvents used for purposes of the present invention may be water, alcohol, and aqueous alcohol solutions, the preferred solvent for purposes of the present invention is water.

The following examples are given to further illustrate the invention as set forth herein. The example, however, is being given primarily for the purposes of illustration and should not be construed as limiting the invention to the details given.

The novel sanitizing, sterilizing and disinfecting composition of the present invention is produced by providing a mixutre of phenol, monopotassium phosphate and disodium phosphate, together with sodium lauryl sulfate and glycerol in amounts to be present on a total weight basis in the final composition of 0.5% phenol, 0.388% monopotassium phosphate and 0.108% disodium phosphate, 0.5% glycerol and 0.25% sodium lauryl phosphate remainder being deionized water as an activating agent composition. This solution is then combined with a 50% aqueous glutaraldehyde solution sold under the Ucarcide brand name in proportions so that the resultant sanitizing, sterilizing and disinfecting composition comprises 0.15% by total weight of glutaraldehyde, 0.5% phenol, 0.0388% monopotassium phosphate, 0.108% disodium phosphate, 0.5% glycerol and 0.25% by total weight sodium lauryl sulfate with the remainder being deionized water.

The previously described sanitizing, sterilizing and disinfecting composition has been found to remain efficacious against bacteria, virus and other microorganisms for a period of up to sixty days. Accordingly, the sanitizing, sterilizing and disinfecting composition made in accordance with the present invention exhibits an extended shelf life and storage stability as compared to conventional glutaraldehyde solutions commercially available.

The present invention finds utility with respect an almost limitless variety of products, including fabric items made from natural and synthetic material, and articles made from plastic materials, such as TYVEX. In this regard, the fabrics which may be used in accordance with the present invention include non-woven and woven fabrics, including those made from natural materials such as paper or other cellulosic materials, cotton, wool, and blends thereof, as well as synthetic materials such as nylon, rayon, acrylics, and the like. It is preferred, however, to use a fabric made from cellulosic materials for purposes of the present invention. The plastics which may be used to form the sheeted, molded or extruded articles incorporating glutaraldehyde in accordance with the present invention may be selected from conventional plastic materials which have been found to be acceptable for use in the delayed-delivery systems. Plastic material which may be used in the present invention include flexible plastics, such as polyvinyl chloride, or low density polyethylene, as well as high density polyethylene fibers, such as spunbonded olefin commercially available under DuPont's trademark TYVEX, in addition to an ethylene-vinyl acetate copolymer resin, such as commerically available Dupont EVA 3185. This ethylene-vinyl acetate copolymer resin exhibits the following physical characteristics: melt index of 43 decigrams/min., ASTM D-1238, weight % vinyl acetate 33, and a density of 960 kg/m$^3$ (0.96 gm/cc ACTM D-792).

The fabric to be treated is preferably cut into sections of any desired size, such as twelve inch squares, which are impregnated with a suitable gluteraldehyde solution. In the embodiment where Sporicidin is used, the Sporicidin is diluted with water. It is preferred to dilute commercial-strength Sporicidin with water in a ratio which falls within the range of 1:12-30. Particularly preferred results are achieved when Sporicidin is diluted with water in the ratio of 1:12.5, 1:16, 1:30. The other glutaraldehyde solutions which have previously been mentioned, including the inventive glutaraldehyde solution, however, may be used without further dilution with water. The sections of fabric may be impregnated with the glutaraldehyde solutions by any means which assures complete saturation of the fabric with the solution, including spraying the fabric or immersing the fabic in a bath of the solution. After impregnation, the sections of fabric may be further reduced in size, separated into individual sheets and packaged in a suitable container which seals the fabric from the atmosphere.

Although any type of container or package which would effectively protect the impregnated fabric from exposure to air from the surrounding environment can be used, plastic films made from polyvinyl chloride, polyethylene and preferably a higfh density polyethylene fiber sheet of spunbonded olefin commerically available under DuPont's TYVEX trademark, and the like which have been processed to render them substantially air-tight or air-impervious are more suitable for this purpose. A plurality of the individually packaged fabric sheets are then assembled in a packet, package or other container for storage, shipping, handling and distribution.

In order to render a surface of an object sporicidal, and/or sanitize, sterilize and disinfect the object, the end user takes one of the individually wrapped sheets of fabric, removes the protective film from the glutaraldehyde-impregnated sheet of fabric and wipes or otherwise contacts the surface of the object which may be contaminated with the Sporicidin-impregnated fabric so as to kill any spores and/or bacteria, virus and other microorganisms which may be present on the surface of the object.

The development of such a disinfectant towelette has overcome many of the inconveniences associated with bulk disinfectants by providing a single-use product which can be used without fear of mistake on the part of te user. Housed within an air tight package in accordance with the present invention, the lifespan of the impregnated towelette exceeds one year.

Related to this, the composition of the present invention may be bottled so that it can be supplied to a separate applicator such as a towel, towelette, swab, sponge or the like for wiping a surface, or the bottle may be provided with a fastened or integral applicator, such as a wick dispenser. In the latter embodiment, all that is required for use is to remove the cap covering the wick applicator from the neck of the bottle and wipe a surface of the applicator over the target area to supply a quantity of the composition suitable for sanitizing, sterilizing and disinfecting in accordance with the present invention. Alternatively, the bottled formulation in accordance with the present invention may be provided with an atomizer or spray device in which case the formulation is applied by spraying the target area with the formulation.

In addition, a number of other articles have been found to be particularly suitable for purposes of the present ivention. For example, a sanitizing wand has been developed which is similar to the previously discussed towelette in terms of application, and also provides the user with a convenient means of always having an instant method of providing self protection from potential carriers of infectious microorganisms. For instance, when using a public telephone, or toilet, the consumer need only rub the mouth and ear piece of the telephone or the seat of the toilet to ensure a high degree of personal safety. Additionally, should a person be conerned when handling small items of common use, he need only rub the sanitizing wand over the article thereby ensuring safety. The applications for this product are numerous and parallel those previously noted in the towelette descriptions.

Infection control containers similar in methodology to the telephone disk, have also been developed which use a specialized form of the active ingredient of the composition in accordance with the present invention. In this instance, however, the finished produce is an extruded bag having a final tensile strength exceeding that of 3 mil. While providing a vapor kill effect upon all contaminant housed within the container. As the removal of contaminants has become a major conern to all, this product should ensure a after environment for those who have the responsibility of removing the waste material. The applications for this product are numerous. As the container may be extruded in a variety of sizes, the infection control containers lend themselves to use in the institution, laboratory, dental and physicians' office, industrial/commerical and retail. The three (3) representative sizes include: (i) 30" x 40" -for removal of large amounts of infectious wastel; (ii) 16" x 20" - to fit wastebaskets, allowing removal of local office generated infectious waste contaminants; and (iii) 6" x 12" - for bedside purposes, allowing removal of patient generated infectious waste, i.e., infected tissues, etc.

The swabs in accordance with the present invention are packaged in a format similar to the towelette and have a size, for example, of 1 1/2" x 2 1/2". The folded woven material of the swab also uses the broad spectrum antimicrobial agent glutaraldehyde as its active ingredient, and merely employing a minute concentration the swab will then ensure that all prep areas are free from contaminants.

In addition to the previously described items, the products of the present invention are intended to include a number of other product types each of which incorporates the aforementioned active ingredient, such as impregnated surgical masks, environmental protection goods, specialized surgical trays and specialized liners for "body-bags", and like products where contamination or risk of contamination is a concern.

Another preferred article made and used in accordance with the present invention is a sanitary attachment for a telephone transmitter or receiver. The concern for providing an effective sanitary attachment for telephones has existed almost since the telephone was first invented. As one can appreciate, the mouthpiece of a telephone is prone to be contaminated with numerous germs as a result of being brought close to the mouth of the user. Accordingly, numerous devices have been developed in an attempt to sanitize, sterilize or otherwise disinfect the mouthpiece of telephones to kill bacteria and germs which may be responsible for illness and diseases, such as the common cold, flu and the like. In recent years, however, there has been a heightened concern for protection against virus, such as AIDS and Herpes, which are believed to be spreading at a rapid rate. Inasmuch as glutaraldehyde solutions such as Sporicidin, are among the few germicidal solutions effective against AIDS and Herpes, the present invention finds particularly utility in the production of articles incorporating gluaraldehyde solutions which have been formed into attachments for a telephone mouthpiece to effectively protect the user from essentially all types of bacterial, virus and other microorganisms.

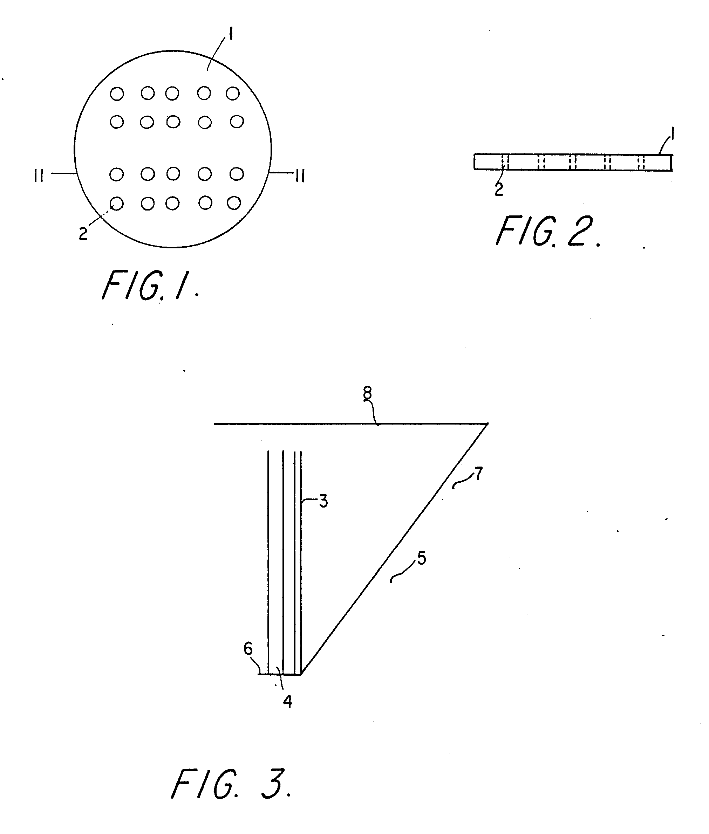

A sanitary telephone attachment made in accordance with the present invention is generally shown in Figs. 1 and 2 in the attached drawings. As shown, the sanitary telephone attachment has been formed in the shape of a disk having a body portion 1 made of plastic material, such as polyvinyl chloride, low density polyethylene, or ethylene-vinyl acetate resin. The disk is shown as being provided with a number of orifices 2 so as not to mute the transmission of sound through the disk, and any number or arrangement of orifices which is effective for this purpose may be provided in the telephone disk of the present invention. Although the plastic material may be sheeted, cut into the desired shape and perforated, it is preferred to extrude the plastic material through a die plate suitable for imparting the desired disk-shape with a suitable arrangement of orifices.

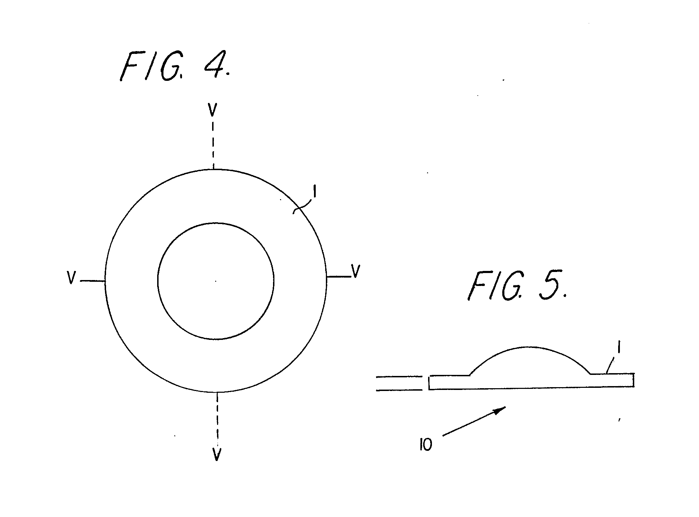

Another embodiment of the telephone disk in accordance with the present invention is shown in Figs. 4 and 5. In this embodiment, the central portion 20 of te body 1 is thicker than the peripheral portion of the disk and is generally convex in shape. An adhesive backing 22 is also provided so that the disk can be easily attached to the transmitter or reciever of a telephone. Although preferred, the convex enlarged central portion of the disk need not be provided with orifices therein as is the case for the embodiment illustrated in Figs. 1 and 2.

Again utilizing glutaraldehyde as the active ingredient in conjunction with a series of other specialized chemicals, in the production of the composition in accordance with the present invention, this sanitary telephone disk item provides a sanitizing environment for common use telephones. Developed into a form that when formulated within a plastic housing, as described herein, causes a blooming effect, this product has a life span of approximately three months. The consumer need only replace the disk to ensure a continued inexpensive approach to the reduction and transmission of telephone transmitted infectious pathogens.

A procedure for producing a sanitary telephone attachment for purposes of the present invention will now be described. Initially, 99 parts of an ethylene-vinyl acetate resin are combined with one part of Sporicidin diluted with water in a ratio of 1:12.5, 1:16, 1.30. In addition, an effective amount of a litmus base indicator was also included in the mixture. The foregoing materials were then tumble-blended without heating for about 5 minutes to achieve optimum dispersion of the solution within the plastic material to produce a base blend or mixture of ethylene-vinyl acetate incorporating Sporicidin. The base blend or mixture was then extruded using a standard one inch single screw extruder exhibiting a heat profile ranging from 250°F at the feed throat to 300°F at the die plate to result with an elongate extrusion or piece. The extruded material was then cooled in a water bath prior to drying to render it suitable for subdividing into pellets. The pellets of ethylene-vinyl acetate resin incorporating Sporicidin solution where then blended with a batch of ethylene-vinyl acetate resin in a ratio of 1:9 to produce and extrudable admixture or mass which was then passed through an extruder and cut into disks having the size and arrangement of orifices as shown in Fig. 1. The sanitary telephone disks incorporating Sporicidin where then individually sealed within two layers of an air-impervious plastic material, such as polyethylene, and assembled into an appropriate packet or other suitable container for shipping, storage, handling and distribution.

A preferred packet for storing a plurality of individually packaged articles made in accordance with the present invention is generally illustrated in Fig. 3. As shown, the individual articles incorporating a glutaraldehyde solution are packaged within air-impervious films 3 preferably made from a sheet of a high density polyethylene film, spunbonded olefin product, commercially available under DuPont's TYVEX trademark. The individual enclosures for the impregnated articles are connected together at one end 4 by glue, staples or merely being heat sealed, in addition to being fastened within a protective packet or container 5 until ready for use. A particularly suitable packet is one similar to a match book cover having generally U-shaped end 6 wrapped around the lower ends 4 of the individual containers for the articles in such a way as to secure them to the packet which also includes a back side 7 and front side 8 adapted to fold over and engage end 6 to protect the plastic film packaged articles from damage.

## EXAMPLES

The following tests were conducted to show the efficacy of the preferred sanitizing, sterilizing and disinfecting solution of the present invention. For puposes of these tests the sanitizing, sterilizing and disinfecting compsition, i.e., inventive composition, was composed of 0.15% glutaraldehyde, 0.5% phenol, 0.0388% monopotassium phosphate, 0.108% disodium phosphate, 0.25% sodium lauryl sulfate, and 0.5% glycerol with the remainer being water. The protocols used in the testing were according to the methods for evaluating disinfectants described in the ADAC manual. Except for Serratia marcescens the bacterial strains used were those recommended by the AOAC and all strains were at least as resistant to phenol as is prescribed in the manual.

Experiments were done using two controls which were necessary to insure that test results were valid.

A viability control was done to verify that the cultures were viable. In this case the organisms were treated in the same manner as test cultures except for exposure to the sanitizing, sterilizing and disinfecting composition of the present invention.

Controls including the sanitizing, sterilizing and disinfecting composition of the present invention were done to confirm that negative (no growth) cultures treated with the sanitizing, sterilizing and disinfecting composition of the present invention were not due to carry over of the disinfectant into the subcultures. To this end test organisms from stock cultures were inoculated into media containing the amount of the sanitizing, sterilizing and disinfecting composition of the present invention carried over during routine sampling. For valid results both controls must be positive for growth.

## TEST FOR BACTERICIDAL ACTIVITY

### Procedure:

Culture tubes were sterilized by autoclaving and 5 ml of the sanitizing, sterilizing and disinfecting composition of the present invention at use dilution was piped into each tube. After equilibrution at 20°C the tubes were inoculated with 0.5 ml of 24 hr cultures of test organism in nutrient broth. At appropriate intervals the tubes containing the sanitizing, sterilizing and disinfecting composition of the present invention were sampled with a sterile 4 mm wire loop and subcultured into nutrient broth. The subcultures were incubated at 37°C for 48 hours and the results recorded as growth (+) or no growth (-).

Results:

TABLE I

| Test Organism[2,3] | Viability Control | Inventive[1] Composition Control | Minutes Contact Time | Growth |
|---|---|---|---|---|
| Pseudomonas aeruginosa ATCC 15442 | + | + | 5 | − |
| Salmonella typhi ATCC 6539 | + | + | 5 | − |
| Staphylococcus aureus ATCC 6538 | + | + | 5 | − |
| Mycobacterium smegmatis LM-15 | + | + | 5 | − |
| Serratia marcescens | + | + | 5 | − |

1. Final concentration of inventive composition was 0.15%.
2. The initial concentration of bacteria were at least 1 x 10^6/ml.
3. The test organisms were ATCC cultures commonly used in testing disinfectants. Thus, P. aeruginosa is a member of the genus Pseudomonas and is among the most resistant vegetative bacteria to chemical disinfectants. Staphylococcus aureus is an important pathogen and is also resistant to disinfectants. Salmonella typhi, a member of the enteric bacteria, is the cause of typhoid fever. The culture used in this study is at least as resistant to disinfectants as the other enterics and thus can serve as an indicator of the effects of disinfectants on the enteric group. M smegmatis is used in presumptive testing of disinfectants because it is easy to culture and grows rapidly. Herpes simplex virus type 1, the cause of the oral form of herpes, is an enveloped DNA virus.

TEST FOR EFFICACY AS A SURFACE DISINFECTANT

Procedure:

Efficacy of the sanitizing, sterilizing and disinfecting composition of the present invention as a surface disinfectant was determined according to the AOAC penicylinder procedure. This procedure involves soaking penicylinders in 24 hr. broth cultures of the test organisms. The cylinders are then dried for 30 minutes and transferred to use dilution solutions of the sanitizing, sterilizing and disinfecting composition of the present invention. At intervals the cylinders are removed and transferred to growth media and incubated 48 hours at 37°C. Results are scored as + or - depending on growth or no growth in the subcultures. Viability controls consisted on soaking impregnated penicylinders in saline and then culturing in growth medium. The control using the sanitizing, sterilizing and disinfecting composition of the present invention consisted of soaking sterile cylinders therein and then transferring the cylinders to subculture media inoculated with the test organisms. Both controls must be positive for a valid test.

## Results:

### TABLE II

| Test Organism[1] | Viability Control | Inventive Composition Control | Minutes Contact Time | Growth |
|---|---|---|---|---|
| Pseudomonas aeruginosa ATCC 15442 | + | + | 5 | − |
| Salmonella typhi ATCC 6539 | + | + | 5 | − |
| Staphylococcus aureus ATCC 6538 | + | + | 5 | − |
| Mycobacterium smegmatis LM-15 | + | + | 5 | − |
| Serratia marcescens | + | + | 5 | − |

1. Penicylinders were soaked in cultures containing at least $1 \times 10^6$ organisms/ml.

DISINFECTION OF OTOSCOPE TIPS TEST

Procedure:
1. Otoscope tips were immersed 24 hours in nutrient broth cultures of the test organisms.
2. The tips were then placed in beakers and dried for 1 hour at 37°C.
3. The dried tips were transferred to sterile beakers and immersed in the sanitizing, sterilizing and disinfecting composition of the present invention.
4. At intervals of 15 and 30 minutes tips were removed and transferred to beakers containing nutrient broth.
5. The beakers were incubated for 48 hours at 37°C. Results were recorded as + or - growth as evidenced by turbidity in the cultures.
6. Controls for the above tests were as follows:
a. In order to insure that the organisms on the tips were viable a contaminated tip was immersed in saline for 20 minutes and then transferred to nutrient broth. This is the viability control and growth is required for a valid test.
b. To insure that negative growth results after exposure to the sanitizing, sterilizing and disinfecting composition of the present invention were not due to carry-over of the disinfectant to the subcultures, a sterile tip was immersed in the sanitizing, sterilizing and disinfecting composition of the present invention for 30 minutes and then transferred to nutrient broth. The culture was then inoculated with the test organism and incubated at 37°C. This is the control using the sanitizing, sterilizing and disinfecting composition of the present invention and growth of the inoculum is required for a valid test.

Results:

TABLE III

| Test Organism | Viability Control | Inventive Composition Control | Minutes Contact Time | |
|---|---|---|---|---|
| | | | 15 | 30 |
| Pseudomonas aeruginosa ATCC 15442 | + | + | − | − |
| Salmonella typhi ATCC 6539 | + | + | − | − |
| Staphylococcus aureus ATCC 6538 | + | + | − | − |
| Mycobacterium smegmatis LM-15 | + | + | − | − |
| Serratia marcescens | + | + | − | − |
| E. coli ATCC 11229 | + | + | − | − |

VIRICIDAL ACTIVITY TEST

Procedure:

Vivicidal acitivity of the sanitizing, sterilizing and disinfecting composition of the present invention was determined using the USEPA surface disinfection procedure. Sterile glass slides were coated with virus suspended in minimal essential medium supplemented with 5% fetal calf serum (no serum for rotavirus). The slides were dried at room temperature with sterile air and then immersed in use dilution sanitizing, sterilizing and disinfecting composition of the present invention for 15 minutes. The slides were removed and the viruses on the slides eluted into saline with a sterile rubber policeman. The saline was appropriately diluted and 0.2 ml aliquots of the dilutions inoculated onto appropriate host cell cultures (vero for HSV and polio, MA104 for rotavirus). After incubation to allow for plaque development the cultures were stained and plaques counted. Controls were done in an identical manner except for exposure to the sanitizing, sterilizing and disinfecting composition of the present invention.

Results:

## TABLE IV

| Dilution of Virus from Surface | Virus Germicide (recovery from treated surface) | | | | | Virus Control (recovery from untreated surface) | | | | | Cytoxicity Control (No virus) | | | |
|---|---|---|---|---|---|---|---|---|---|---|---|---|---|---|
| Slide No. | 1 | 2 | 3 | 4 | 5 | 1 | 2 | 3 | 4 | 5 | 1 | 2 | 3 | 4 |
| $10^{-1}$ | T | T | T | T | T | + | + | + | + | + | T | T | T | T |
| $10^{-2}$ | 0 | 0 | 0 | 0 | 0 | + | + | + | + | + | 0 | 0 | 0 | 0 |
| $10^{-3}$ | 0 | 0 | 0 | 0 | 0 | + | + | + | + | + | 0 | 0 | 0 | 0 |
| $10^{-4}$ | 0 | 0 | 0 | 0 | 0 | + | + | + | + | + | 0 | 0 | 0 | 0 |
| $10^{-5}$ | 0 | 0 | 0 | 0 | 0 | + | + | + | + | + | 0 | 0 | 0 | 0 |
| $10^{-6}$ | 0 | 0 | – | – | – | + | + | 0 | 0 | 0 | 0 | 0 | 0 | 0 |

Virus Recovered: $10^5$

Toxicity: $10^1$

Virus Reduction: $10^4$ or more

## TABLE IVB

| Dilution of Virus from Surface | Virus Germicide (recovery from treated surface) | | | | Virus Control (recovery from untreated surface) | | | | Cytoxicity Control (No virus) | | | |
|---|---|---|---|---|---|---|---|---|---|---|---|---|
| Slide No. | 1 | 2 | 3 | 4 | 1 | 2 | 3 | 4 | 1 | 2 | 3 | 4 |
| $10^{-1}$ | T | T | 0 | 0 | + | + | + | + | T | T | 0 | 0 |
| $10^{-2}$ | 0 | 0 | 0 | 0 | + | + | + | + | 0 | 0 | 0 | 0 |
| $10^{-3}$ | 0 | 0 | 0 | 0 | + | + | + | + | 0 | 0 | 0 | 0 |
| $10^{-4}$ | 0 | 0 | 0 | 0 | + | + | + | + | 0 | 0 | 0 | 0 |
| $10^{-5}$ | 0 | 0 | 0 | 0 | + | + | + | + | 0 | 0 | 0 | 0 |
| $10^{-6}$ | | | | | | | | | | | | |

Virus Recovered:    $10^5$
Toxicity:           $\pm\, 10^{-1}$
Virus Reduction:    $10^4$ or more

## TABLE IVC

| Dilution of Virus from Surface | Virus Germicide (recovery from treated surface) | | | | | Virus Control (recovery from untreated surface) | | | | | Cytoxicity Control (No virus) | | | |
|---|---|---|---|---|---|---|---|---|---|---|---|---|---|---|
| Slide No. | 1 | 2 | 3 | 4 | 5 | 1 | 2 | 3 | 4 | 5 | 1 | 2 | 3 | 4 |
| $10^{-1}$ | T | T | T | T | T | + | + | + | + | + | T | T | T | T |
| $10^{-2}$ | 0 | 0 | 0 | 0 | 0 | + | + | + | + | + | 0 | 0 | 0 | 0 |
| $10^{-3}$ | 0 | 0 | 0 | 0 | 0 | + | + | + | + | + | 0 | 0 | 0 | 0 |
| $10^{-4}$ | 0 | 0 | 0 | 0 | 0 | + | + | + | + | + | 0 | 0 | 0 | 0 |
| $10^{-5}$ | 0 | 0 | 0 | 0 | 0 | + | + | + | + | + | 0 | 0 | 0 | 0 |
| $10^{-6}$ | 0 | 0 | 0 | 0 | 0 | + | 0 | 0 | + | + | 0 | 0 | 0 | 0 |

```
Virus Recovered:   10^5 - 10^6
Toxicity:          10^1
Virus Reduction:   10^4 or more
```

For the data tabulated in Tables IVA-C:

T = Toxicity

+. = Viral Cytopathic Effect

0 = No Cytopathic Effect

Each number represents a separate slide study.

TEST FOR EFFECT OF TIME ON BACTERICIDAL ACTIVITY

Procedure:
A solution of the sanitizing, sterilizing and disinfecting composition of the present invention was freshly formulated and stored in a brown glass container at ambient room temperature. At intervals the solution was sampled and cidal activity determined against Pseudomonas aeruginosa. This organism was chosen for these experiments since in these tests it was the most resistant organism. The procedure for testing efficacy was the same as the use dilution test described in the AOAC manual. In the final testing of bactericidal activity of 70 day old sanitizing, sterilizing and disinfecting composition of the present invention the complete panel of bacteria were used.

16

Results:

TABLE VA

| Age of inventive composition in bags | Viability Control | Minutes contact time | | |
|---|---|---|---|---|
| | | 5 | 10 | 15 |
| 0 | + | − | − | − |
| 14 | + | − | − | − |
| 28 | + | − | − | − |
| 35 | + | − | − | − |
| 42 | + | − | − | − |
| 49 | + | − | − | − |
| 56 | + | − | − | − |
| 63 | + | − | − | − |

1.  Test organism was P. aeruginosa.  Viability control must be positive for a valid test.

TABLE VA

| Test organism | Viability Control | Minutes contact time | | |
|---|---|---|---|---|
| | | 5 | 10 | 30 |
| P. aeruginosa ATCC 15442 | + | + | − | − |
| S. typhi       ATCC 6539 | + | − | − | − |
| S. aureus      ATCC 6538 | + | − | − | − |
| M. smegmatis   LM-15 | + | − | − | − |
| S. Marcescens | + | − | − | − |

1.  Solution of inventive composition was 70 days old.

The foregoing tests indicate that at the recommended use dilution the sanitizing, sterilizing and disinfecting composition of the present invention is an effective broad spectrum disinfectant with bactericidal and viricidal

**0 279 704**

activity. Both gram + and gram - bacteria were equally sensitive to the sanitizing, sterilizing and disinfecting composition of the present invention. Enveloped and non-enveloped viruses were sensitive to the sanitizing, sterilizing and disinfecting composition of the present invention. The sanitizing, sterilizing and disinfecting composition of the present invention is an effective surface disinfectant as indicated by the AOAC and EPA test procedures. The sanitizing, sterilizing and disinfecting composition of the present invention has excellent shelf stability with no loss of cidal activity after 60 days.

From the foregoing description, one skilled in the art can easily ascertain the essential characteristics of this invention and, without departing from the spirit and scope thereof, can made various changes and modifications of the invention to adapt it to various usages and conditions.

**Claims**

1. A composition of activating dialdehyde solutions in the production of sanitizing, sterilizing and disinfecting compositions comprising:
phenol and at least one alkali metal salt of phosphoric acid.

2. The composition in accordance with claim 1, further comprising at least one surfactant and at least one humectant.

3. The composition in accordance with claim 1 or 2, wherein said at least one alkali metal salt of phosphoric acid is a member selected from potassium phosphates, sodium phosphates, and mixtures of potassium phosphate and sodium phosphate.

4. The composition in accordance with claim 3, wherein said member is selected from monopotassium phosphate and disodium phosphate.

5. The composition in accordance with claim 4, wherein said at least one alkali metal salt of phosphoric acid is a mixture of monopotassium phosphate and disodium phosphate.

6. The composition in accordance with claim 2, wherein said surfactant is sodium lauryl sulfate.

7. The composition in accordance with claim 6 or claim 7, wherein said humectant is glycerol.

8. A composition according to claim 2 consisting essentially of phenol, monopotassium phosphate, disodium phosphate, sodium lauryl sulfate, glycerol and water.

9. A sanitizing, sterilizing and disinfecting composition comprising:
a dialdehyde solution, and a composition according to any of claims 1 to 8.

10. The sanitizing, sterilizing and disinfecting composition in accordance with claim 9, wherein said dialdehyde solution is an aqueous glutaraldehyde solution.

11. The sanitizing, sterilizing and disinfecting composition in accordance with claim 10, wherein said glutaraldehyde solution is a member selected from a 25% aqueous glutaraldehyde solution and a 50% aqueous glutaraldehyde solution.

12. The sanitizing, sterilizing and disinfecting composition in accordance with claim 11, wherein said aqueous glutaraldehyde solution is present in sufficient amounts to provide glutaraldehyde within the range of 0.05-0.5%, phenol is present within the range of 0.25-0.75%, monopotassium phosphate is present within the range of 0.01-0.075%, disodium phosphate is present within the range of 0.5-0.12%, sodium lauryl sulfate is present within the range of 0.25-0.75% with the remainder being water.

13. The sanitizing, sterilizing and disinfecting composition of claim 12, wherein said glutaraldehyde is present within the range of 0.075-0.3%, said phenol is present in the amount of about 0.5%, said monopotassium phosphate is present in the amount of about 0.0388%, said disodium phosphate is present in the amount of about 0.108%, said sodium lauryl sulfate is present in an amount of about 0.25%, and said glycerol is present in the amount of about 0.5%.

14. A sanitizing, sterilizing and disinfecting composition consisting essentially of 0.15% glutaraldehyde, 0.5% phenol, 0.0388% monopotassium phosphate, 0.105% disodium phosphate, 0.25% sodium lauryl sulfate, and 0.5% glycerol with the remainder being water.

15. A method for imparting sanitizing, sterilizing and disinfecting properties to fabrics comprising:
a) impregnating a fabric with a composition according to any of claims 10-14; and
b) packaging said fabric in an air-impervious container until ready for use.

16. The method in accordance with claim 15, wherein said solution includes an effective amount of litmus base indicator.

17. A packaged article having sanitizing, sterilizing and disinfecting properties comprising fabric impregnated by a method according to claim 15 or claim 16 sealed within an air-impervious enclosure.

18. A process for sanitizing, sterilizing and disinfecting a contaminated surface comprising:
providing a tangible article according to claim 17 comprising fabric made from polymeric material;
removing said impregnated tangible article from said enclosure; and
applying said impregnated tangible article to said surface so as to expose microorganisms on said surface to the action of said glutaraldehyde.

19. The process of claim 18, wherein said tangible article is selected from appliances, applicators, bags, bedding, cloths, containers, coverings, clothes, equipment, fabrics, flooring, garments, instruments, liners, masks, tissues, towels, towelettes, trays, sponges, swabs, and utensils.

18

20. The process of claim 19, wherein said tangible article is an attachment adapted to be mounted to a telephone receiver or mouthpiece.

21. An attachment adapted to be applied to a receiver or mouthpiece of telecommunication equipment comprising a body portion of plastic material incorporating a sanitizing composition according to any of claims 10 to 14.

22. The attachement of claim 21, wherein said body portion further incorporates a litmus base indicator.

23. The attachment of claim 21, comprising a removable air-impervious film sealed around said body portion.

24. A method for producing a sanitizing, sterilizing and disinfecting glutaraldehyde solution having extended shelf life comprising:

a) providing a composition including phenol, monopotassium phosphate, disodium phosphate, sodium lauryl sulfate, glycerol and water;

b) mixing glutaraldehyde solution with said composition to result with a sanitizing, sterilizing and disinfecting glutaraldehyde solution comprising:

i) 0.15% by total weight glutaraldehyde;

ii) 0.5% by total weight phenol;

iii) 0.0388% by total weight monopotassium phosphate;

iv) 0.108% by total weight disodium phosphate;

v) 0.25% by total weight sodium lauryl sulfate;

vii) the balance being solvent.

FIG. 1.

FIG. 2.

FIG. 3.

20·05·88

0279704

FIG. 4.

FIG. 5.